# EUROPEAN PATENT SPECIFICATION

(11) **EP 1 299 083 B1**
(45) Date of publication and mention of the grant of the patent: **27.04.2005**
(21) Application number: 01942107.2
(22) Date of filing: 07.06.2001
(51) Int. Cl.: A61K 9/00, A61K 47/38, A61L 31/04, A61L 17/10

(54) **IMPLANTABLE LOW MOLECULAR WEIGHT CELLULOSIC COMPOSITIONS**
IMPLANTIERBARE ZUSAMMENSETZUNGEN ENTHALTEND NIEDERMOLECULARE ZELLULOSEDERIVATE
COMPOSITIONS IMPLANTABLES DE CELLULOSES DE FAIBLE POIDS MOLECULAIRE

(30) Priority: 09.06.2000 US 591373
(43) Date of publication of application: 09.04.2003
(73) Proprietor: GENZYME CORPORATION, Cambridge, Massachusetts 02139 (US)
(72) Inventor: BURNS, James, W., Watertown, MA 02171 (US); MILLER, Robert J., Quincy, MA 02169 (US)
(74) Representative: Wallace, Sheila Jane
(86) International application number: PCT/US2001/018592
(87) International publication number: WO 2001/095875

(56) References cited:
- EP-A- 0 815 879
- WO-A-92/20349
- GUIMARAES M A M ET AL: "Urinary excretion of sulfated polysaccharides administered to Wistar rats suggests a renal permselectivity to these polymers based on molecular size" BBA - GENERAL SUBJECTS, ELSEVIER SCIENCE PUBLISHERS, NL, vol. 1335, no. 1-2, 17 April 1997 (1997-04-17), pages 161-172, XP001041029 ISSN: 0304-4165

## Description

### Background of the Invention

This invention relates to low molecular weight carboxymethylcellulose compositions that are absorbed and substantially cleared from the body of a subject within 30 days of the date of implantation in the body. The low molecular weight carboxymethylcellulose compositions of this invention can be safely used in a wide variety of medical applications, such as in medical implants and sustained release pharmaceutical delivery vehicles.

Polyanionic polysaccharides, such as carboxymethylcellulose ("CMC"), carboxymethyl amylose, and hydroxyethyl cellulose, are useful in a wide variety of medical applications, such as in drug delivery and the prevention of surgical adhesion formation. Such polysaccharides can readily dissolve in water to form viscous fluids.

Biocompatible polymers have been commonly used to manufacture implantable medical devices. For such devices, the reabsorption of the polymer, and its subsequent clearance from the body, should be completed within 30 days in order for implants fabricated from the polymeric composition to be designated as short term implants under current FDA regulations. These regulations draw a clear distinction, in terms of compliance, between short term and long term implant devices, and require significantly greater regulatory scrutiny for long term implants. This scrutiny is based primarily on the safety requirements for devices that are intended to remain within the body for a prolonged period of time.

Hyaluronic Acid ("HA") and CMC, in chemically modified (derivatized or cross-linked) forms, are useful as surgical aids to prevent adhesions or accretions of body tissues during the post-operation period. The derivatized HA gel or film is injected or inserted into the locus between the tissues that are to be kept separate to inhibit their mutual adhesion. Chemically modified HA can also be useful for controlled release drug delivery. See U.S. Patent No. 4,937,270 and U.S. Patent 5,017,229 that disclose derivatized versions of HA prepared, for instance, by reacting HA with a carbodiimide. The clearance from the body of these polymers is dependent on the molecular weight of the chemically modified and/or cross-linked polymers. The polymers clear from the blood by glomerulus filtration in the kidneys.

U.S. Patent No. 6, 030,958 describes methods for making water insoluble CMC compositions by reacting the CMC with a carbodiimide, such as EDC. The compositions can be formed into water insoluble gels which can also contain drug substances. The molecular weight of the CMC used to prepare these compositions is described as being in the range of from 9 x 10⁴ daltons to 3 x 10⁶ daltons.

EP-A-0815879 discloses bioabsorbable medical devices prepared by oxidising derivatives of cellulose, including methyl cellulose, CMC and cellulose acetate. The resultant modified cellulose derivatives can be broken down into low molecular weight fragments that can be excreted through the kidneys.

Elkins et al., *Adhesion Prevention by Solutions of Sodium Carboxymethylcellulose in the Rat*, Fertility and Sterility (1984), describe the residence time of solutions of sodium carboxymethylcellulose and dextrose in the peritoneal cavity of rats. These materials were evaluated for effectiveness against post operative adhesions. The sodium carboxymethylcellulose solutions had molecular weights of up to about 350,000 daltons.

Cellulose, and derivatives of cellulose, are very resistant chemically to hydrolytic depolymerization. CMC is prepared by reacting an alkali cellulose and sodium chloroacetate under very basic conditions at elevated temperatures. Carboxymethylcellulose, in chemically unmodified form, is water soluble and disperses readily in water.

Cellulose and cellulose derivatives can be enzymatically depolymerized by a variety of non-mammalian organisms. A variety of bacterial species have been shown to depolymerize CMC using β-glucanases. See Sierks, M.R., et al., *Appl. Environ. Microbiol*. 50 (3), 634, (1985); Lamot, E., et al, *Z. Allg. Mikrobiol*. 19, (5), 345, (1976); C. G. van Ginkel et al, *Environ. Tox. Chem*., 15, (3), 270, (1996). Fungi also possess the enzymes required for depolymerization of cellulose and CMC, most notably the fungi responsible for wood rot. See Almin, K.E., et al, *Eur. J. Biochem.* 51, (1), 207, (1975); and Kanda, T, et al, *J. Biochem.(Tokyo)*, 84, (5), 1217, (1978).

Mammalian systems cannot degrade cellulose because they do not possess the degradative enzymes found in some bacteria and fungi. Therefore, cellulose and cellulose derivatives will not easily degrade when implanted or ingested by mammals. Since catabolic pathways for the degradation of cellulose and cellulose derivatives are not available to mammals, cellulose and cellulose derivatives must clear by water solubility and passage through the kidneys.

The clearance of an exogenous drug, solute, or polymer in a mammal occurs after the material enters the circulatory system. Once in the circulatory system, the exogenous materials are eliminated from the body by either hepatic metabolism, biliary excretion or renal filtration. Clearance by the liver is controlled by several factors such as blood flow to the liver, binding of the solute to serum proteins, and whether or not the solute binds to hepatic enzymes or binding sites. See *Cecil Textbook of Medicine*; Wyngaarden, J.B., and Smith, L.H., Jr., Eds; W.B. Saunders Company, Philadelphia, 1982, pp. 50. Since the degradative enzymes for cellulose and cellulose derivatives are peculiar to bacteria and fungi, cellulose and cellulose derivatives will not bind to any of the liver enzymes in mammals, and in turn will not clear through the liver. Therefore, the predominate route of clearance of these polysaccharides is by renal filtration, more specifically by glomerular filtration.

The passage of a solute through the kidneys is controlled by passage through the glomerulus. The rate and ease of transport through the glomerulus is in turn controlled by the molecular weight, charge and Stokes-Einstein Radius of the solute. See Oliver, J.D., et al., *Bull. Math. Biol*., 56, (3), 369, (1994). The structure of the glomerulus is comprised of a meshwork of type IV collagen fibrils and negatively charged heparin-sulfate proteoglycans See Langer, K.H., *Klin. Wochen*. 63, (18), 835, (1985). The anionic charge of the glomerular membrane excludes negatively charged macromolecules such as scrum albumin. The ability of uncharged macromolecules to undergo glomerular filtration has been reported in the literature. Uncharged macromolecules between 40,000 daltons to 80,000 daltons, such as dextran and polyvinylpyrrolidone, readily pass through the glomerulus. See Alt, J.M. et al., *Con. Nephrol*. 19, 217, (1980). For any given size, negatively charged macromolecules have been shown to not readily cross the glomerular wall. See Brenner, B.M., et al. *Am. J. Physiol*. 234, (6), F455, (1978).

Accordingly, one skilled in the art would not expect negatively charged cellulose derivatives, such as carboxymethylcellulose, to clear from mammals through the kidneys since the glomerular membrane would tend to exclude negatively charged species. Moreover, the absence of an enzyme for degrading cellulose derivatives in mammals would prevent clearance through the liver.

### Summary of the Invention

The present invention features low molecular weight, cellulose compositions that are used to prepare medical implants, devices and pharmaceutical delivery vehicles. The cellulose compositions of this invention are unmodified, and are capable of being reabsorbed in the body of a subject within 30 days of the date of implantation. Typical cellulose compositions include those based on carboxymethylcellulose and hydroxyethyl cellulose.

Surprisingly, it has been found that CMC compositions having a molecular weight of less than about 100 kdaltons, preferably less than about 85 kdaltons, and most preferably less than about 75 kdaltons, are capable of being fully reabsorbed and excreted from the body of a subject. CMC compositions having a molecular weight exceeding 100 kdaltons are generally not capable of being reabsorbed by the body within 30 days, and thus do not satisfy current FDA regulations for short term implants. This correlation between the molecular weight of CMC and the ability of the body to absorb the CMC had not been previously known in the art.

Various implant devices can be prepared with the CMC compositions of this invention, including, but not limited to, prosthetic devices, typically synthetic or bioprosthetic devices, stents, grafts, sutures, catheters, tubings, guidewires, and the like. These devices can be installed in the body using routine surgical procedures. Examples of routine surgical implantation are subcutaneous, intramuscular, or intravenous injection through a needle or canula, and placement of the implant in the body after laparotomy or endoscopy.

The CMC compositions of this invention can also be used as implantable drug delivery devices. In this embodiment, the drug substance is dispersed within the CMC polymer matrix. The composition can then be molded or extruded into any desired shape for implantation in the body. Suitable drug substances include proteins, such as growth factors and enzymes, pharmaceuticals, antibodies, biopolymers, and biologically compatible synthetic polymers. The CMC composition can be formulated to provide for specific time release characteristics to maximize the effectiveness of the delivered drug.

In another embodiment, the invention embraces a method for preparing an implant device by selecting an unmodified cellulose composition having a molecular weight of less than 100 kdaltons, selecting a therapeutic drug substance for use in treating a patient having a particular medical condition requiring treatment, and combining the drug substance and cellulose composition to prepare the implant device. Preferably, the cellulose composition has a molecular weight of less than about 85 kdaltons, and more preferably less than 75 kdaltons. The drug substance and cellulose composition can be physically combined by mixing or blending the components to form the implant device. The drug release characteristics of the device will depend on the relative proportion of the components, the type and solubility of the polymer selected, and the particle size and distribution of the drug substance in the device.

This invention is also intended to encompass, unless defined otherwise, all technical and scientific terms used herein have the same meaning as commonly understood by one of ordinary skill in the art to which this invention belongs. Although any method and materials similar or equivalent to those described herein can be used in the practice or testing of the present invention, the preferred methods and materials are now described. Unless mentioned otherwise, the techniques employed or contemplated herein are standard methodologies well known to one of ordinary skill in the art. The materials, methods and examples are illustrative only and not intended to be limiting.

Other features and advantages of the invention will be apparent from the following description of the preferred embodiments, and from the, claims.

### Brief Description of the Drawings

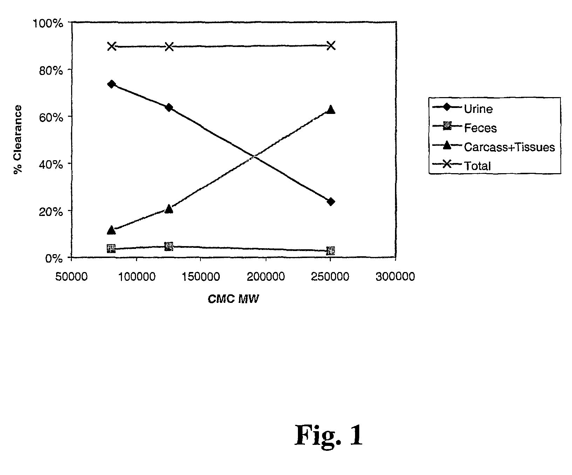

Figure 1 is a graphical representation of the amount of radioactively labeled CMC that clears the body as a function of the starting polymer molecular weight.

### Detailed Description of the Invention

The cellulose compositions of the present invention are unmodified versions of cellulose. Particular cellulose compounds which are useful in the practice of the invention include carboaymethycellulose, carboxymethyl amylose and hydroxyethyl cellulose. The cellulose compositions can also include additives, other polymers, and in some applications, drug substances.

The CMC compositions of this invention should also be biocompatible. A "biocompatible" substance, as that term is used herein, is one that has no medically unacceptable toxic or injurious effects on the biological function of the subject.

Carboxymethylcellulose is a polyanionic polysaccharide containing at least one negatively charged group in which the -CH₂COOH groups are substituted on the glucose units of the cellulose chain through an ether linkage. In general, CMC can be prepared, for instance, by the reaction of an alkali cellulose and sodium chloroacetate. The degree of solubility of the carboxymethylcellulose depends on the degrees of substitution of the carboxymethyl groups on the glucose units. Similarly , the degree of solubility of other cellulose compounds of the invention also depends on the degree of substitution of such compounds. Similarly, the degree of solubility of other cellulose compounds of this invention also depends on the degree of substitution on the compound.

The term "cellulose," as used herein, is intended to indicate unmodified versions of carboxymethylcellulose, carboxymethyl amylose, and hydroxyethyl cellulose. It is intended to denote polymers having various degrees of water solubility, as well as unmodified versions as provided herein.

The term "subject" as used herein is intended to cover any mammal, such as a pig, horse, cow, goat, and the like, but preferably refers to a human.

The CMC composition can be terminally sterilized by autoclaving the composition, and this procedure does not have any adverse impact on the structure of the polymer. Terminal sterilization is a cost effective method for manufacturing the implantable device or drug delivery vehicle, since it has a lower bioburden than aseptic processing, and thereby reduces the risk of infection due to the presence of the implant. Typically, terminal sterilization involves steam autoclaving of the aqueous preparation.

The CMC composition used in the present invention can either be water soluble or insoluble. By the term "water insoluble" is generally meant that the polymer remains intact when implanted in the body of a subject, i.e. in a general aqueous environment, for at least 7 days, and is completely absorbed by the body within 30 days. More precisely, the "water insoluble" CMC compositions of this invention are compositions which, when formed into a solid using a 1% aqueous solution of CMC, modified according to the invention, having dimensions 3 cm x 3 cm x 0.3 mm, and allowed to stand without stirring in a beaker of 50 ml of distilled water at 20°C, remains structurally intact after 20 minutes, with the structural boundaries and edges of the material still being present after 24 hours. Thus, the CMC compositions of this invention are both water soluble and insoluble, as well as being biocompatible.

The carboxymethylcellulose composition of this invention can also include other biocompatible polymers, such as other polyanionic polysaccharides, and more particularly hyaluronic acid. The other polymers can be present in amounts that impart beneficial properties to the composition without being detrimental to the use of the CMC as an implant or drug delivery device. Such properties include improved anti-fouling and anti-platelet adhesion activation characteristics. The term "platelet adhesion", as used herein, means the amassing or aggregation of platelets onto the surface of an implanted device (e.g., a vascular wall, prosthetic device) through interactions of the platelets with the surface, and a device which has "anti-platelet adhesion" characteristics prevents such amassing.

The CMC compositions of this invention can be used to prepare medical implant devices. The compositions may be molded into any desired shape and implanted in the body of a subject. Injection molding is a useful technique for this purpose. Suitable medical implant devices include prosthetic devices, synthetic or bioprosthetic devices, stents, grafts, sutures, catheters, tubings, and guidewires.

The CMC compositions of this invention can also be used to prepare implantable drug delivery devices. Such devices are primarily useful for delivering a variety of drugs to the subject, including proteins, such as growth factors or enzymes, pharmaceuticals, antibodies, biopolymers, and biologically compatible synthetic polymers. The drug delivery device can be formulated so as to provide for the time release of the drug substance. Other additives and excipients which are known in the art can also be included in the CMC composition and incorporated into the drug delivery device formulation, including polyetheylene glycol, polyvinylpyrrolidone, dextran, dextran sulfate, serum albumin, sorbitol, mannitol, and the like.

From the above description, one skilled in the art can readily ascertain the essential characteristics of the present invention, and without departing from the spirit and scope thereof, can make various changes and modifications of the invention to adapt it to various usages and conditions.

As one skilled in the art will appreciate, the compositions of this invention can be made using methods that may differ in certain particulars from those methods exemplified herein.

The following examples of the invention are provided by way of illustration only, and are not intended to limit the invention except as set forth in the appended claims.

### EXAMPLE 1

This example describes the preparation of radiolabeled CMC compositions.

A radiolabeled carboxymethylcellulose solution, in the 125 kdalton to 250 kdalton range, was prepared by reacting C-1-[C14]-2-chloroacetic acid with cotton linter in anhydrous isopropyl alcohol under basic conditions with heating. The resulting solid was isolated by washing with excess isopropyl alcohol and drying under reduced pressure. This yielded an off-white powder that was highly water soluble. The molecular weight of the reaction product was analyzed and formed to have a weight averaged molecular weight of 250 kdaltons by SEC-MALLS analysis.

Radiolabeled CMC with a weight averaged molecular weight of 125 kdaltons was obtained by aging the higher molecular weight CMC for approximately two years at 2°C to 8°C. Radiolabeled CMC with a weight averaged molecular weight of 70 kdaltons was purchased from New England Nuclear Corp., Billerica, Massachusetts.

### EXAMPLE 2

This example describes the molecular weight analysis of radiolabeled CMC.

Molecular weight analysis was performed using an HPLC that had tandem SEC columns (TSK G6000PW and G4000PW). The mobile phase was 0.05 M Na₂SO₄ aqueous, the pH was 9 at a flow rate of 0.6 ml/min. and the injection volume was 100 µL. The molecular weight was determined using a multiangle laser light scattering detector (Wyatt Technologies), coupled with a refractive index detector (Hewlett-Packard).

To determine radioactive purity, the fractions from the HPLC were collected (from 10 ml to 20 ml (>40 kDa), and from 20 ml to 31 ml (<40 kDa)) and analyzed for radioactivity on a liquid scintillation spectrophotometer (Model TRI-CARB 1500, Packard). One ml from fraction 10-20 (sample), and one ml from fraction 20-31 (solvent) were collected and mixed with 10 ml of scintillation fluid (Biodegradable Counting Sensalator Lot A3426, Amersham). The mobile phase was used as the control.

The results of the molecular weight analysis of the C14-labeled CMC used in the clearance studies described in Example 3 are shown in Table 1 below:

**Table 1**

| Group | Molecular Weight by SEC-MALLS |
|---|---|
| Low MW CMC | 80 kdaltons |
| Medium MW CMC | 120 kdaltons |
| High MW CMC | 250 kdaltons |

### EXAMPLE 3

This example describes CMC clearance studies in rats.

C14-labeled CMC, prepared as described above, was dissolved in succinate buffered saline solution, at a pH of 4.0, and at concentrations of 0.5% w/v, with a specific activity of 10-15 µCi/mL. Each animal received between 25 to 40 µCi of radioactive CMC by IP injection. Radioactivity was analyzed in the urine, feces, residuals in the cage, organs and remaining carcass of the animal for up to 30 days post-injection. Figure 1 shows the amount of radioactivity that clears from the body as a function of the CMC starting molecular weight. As shown in Figure 1, the distribution of C14-labeled CMC in rats after 30 days is a function of the molecular weight of the polymer, with the maximum clearance from the body occurring at the lower molecular weight ranges of the polymer.

## Claims

1. A medical implant, device, or implantable pharmaceutical preparation comprising an unmodified carboxymethylcellulose, carboxymethyl amylose or hydroxyethyl cellulose composition having a molecular weight of less than 100 kdaltons.

2. A medical implant, device, or implantable pharmaceutical preparation according to claim 1 in which the composition has a molecular weight of less than 85 kdaltons.

3. A medical implant, device, or implantable pharmaceutical preparation according to claim 2 in which the composition has a molecular weight of less than 75 kdaltons.

4. A medical implant, device, or implantable pharmaceutical preparation according to any one of the preceding claims wherein the unmodified cellulose composition is hydroxyethyl cellulose.

5. A medical implant, device, or implantable pharmaceutical preparation according to any one of claims 1 to 3 wherein the unmodified cellulose composition is carboxymethylcellulose.

6. A medical implant, device, or implantable pharmaceutical preparation according to any one of the preceding claims wherein the composition includes at least one other polymer.

7. A medical implant, device, or implantable pharmaceutical preparation according to claim 6 wherein the at least one other polymer is hyaluronic acid.

8. A medical implant, device, or implantable pharmaceutical preparation according to any one of the preceding claims which is a prosthetic device, a synthetic or bioprosthetic device, a suture, or a tubing.

9. A medical implant, device or implantable pharmaceutical preparation according to any one of the preceding claims wherein the device is used to deliver a drug substance selected from the group consisting of proteins, pharmaceuticals, antibodies, biopolymers, and biologically compatible synthetic polymers.

10. A medical implant, device or implantable pharmaceutical preparation according to claim 9 wherein the protein is a growth factor or an enzyme.

11. A method for preparing an implant device comprising
selecting a drug substance for use in treating a patient suffering from a particular medical condition,
selecting an unmodified carboxymethylcellulose, carboxymethyl amylose or hydroxyethyl cellulose composition having a molecular weight of less than 100 kdaltons which, upon implantation in a subject as a component of an implant device, is capable of releasing the drug in the patient according to a predetermined release profile, and
preparing an implant device by combining the drug substance and the cellulose composition.

12. The method of claim 11 additionally including any of the features of claims 2 to 7.

## Patentansprüche

1. Medizinisches Implantat, Vorrichtung oder implantierbares pharmazeutisches Präparat umfassend eine nicht modifizierte Carboxymethylcellulose, Carboxymethylamylose oder Hydroxyethylcellulose-Verbindung mit einem Molekulargewicht von weniger als 100 kdaltons.

2. Medizinisches Implantat, Vorrichtung oder implantierbares pharmazeutisches Präparat nach Anspruch 1, wobei die Verbindung ein Molekulargewicht von weniger als 85 kdaltons aufweist.

3. Medizinisches implantat, Vorrichtung oder implantierbares pharmazeutisches. Präparat nach Anspruch 2, wobei die Verbindung ein Molekulargewicht von weniger als 75 kdaltons aufweist.

4. Medizinisches Implantat, Vorrichtung oder implantierbares pharmazeutisches Präparat nach einem der vorstehenden Ansprüche, wobei die nicht modifizierte Celluloseverbindung Hydroxyethylcellulose ist.

5. Medizinisches Implantat, Vorrichtung oder implantierbares pharmazeutisches Präparat nach einem der Ansprüche 1 bis 3, wobei die nicht modifizierte Celluloseverbindung Carboxymethylcellulose ist.

6. Medizinisches Implantat, Vorrichtung oder implantierbares pharmazeutisches Präparat nach einem der vorstehenden Ansprüche, wobei die Verbindung wenigstens ein anderes Polymer enthält.

7. Medizinisches Implantat, Vorrichtung oder implantierbares pharmazeutisches Präparat nach Anspruch 6, wobei das wenigstens eine andere Polymer Hyaluronsäure ist.

8. Medizinisches Implantat, Vorrichtung oder implantierbares pharmazeutisches Präparat nach einem der vorstehenden Ansprüche, das eine prosthetische Vorrichtung, eine synthetische oder bioprosthetische Vorrichtung, ein Nahtmaterial oder ein Röhrchenmaterial ist.

9. Medizinisches Implantat, Vorrichtung oder implantierbares pharmazeutisches Präparat nach einem der vorstehenden Ansprüche, wobei die Vorrichtung dazu benutzt wird ein Medikament ausgewählt aus der Gruppe bestehend aus Proteinen, Pharmazeutika, Antikörpern, biopolymeren und biologisch kompatiblen synthetischen Polymeren.

10. Medizinisches Implantat, Vorrichtung oder implantierbares pharmazeutisches Präparat nach Anspruch 9, wobei das Protein ein Wachstumsfaktor oder ein Enzym ist.

11. Verfahren zur Herstellung einer implantierbaren Vorrichtung umfassend
Auswählen eines Medikamentes zur Behandlung eines Patienten, der an einem bestimmten medizinischen Zustand leidet,
Auswählen einer nicht modifizierten Carboxymethylcellulose, Carboxymethylamylose oder Hydroxyethylcllulose Verbindung mit einem Molekulargewicht von weniger als 100 kdaltons, die, nach der Implantation in einem Subjekt als eine Komponente einer implatierbaren Vorrichtung, geeignet ist das Medikament in den Patienten entsprechend einem vorgegebenen Abgabeprofil abzugeben und
Herstellen einer implantierbaren Vorrichtung durch Kombination eines Medikamentes und der Cellulose Verbindung.

12. Verfahren nach Anspruch 11, das zusätzlich die Merkmale der Ansprüche 2 bis 7 aufweist.

## Revendications

1. Implant médical, dispositif, ou préparation pharmaceutique implantable comprenant une composition de carboxyméthyl cellulose, carboxyméthyl amylose ou hydroxyéthyl cellulose non modifiée ayant un poids moléculaire inférieur à 100 kdaltons.

2. Implant médical, dispositif, ou préparation pharmaceutique implantable selon la revendication 1, dans lequel la composition a un poids moléculaire inférieur à 85 kdaltons.

3. Implant médical, dispositif, ou préparation pharmaceutique implantable selon la revendication 2, dans lequel la composition a un poids moléculaire inférieur à 75 kdaltons.

4. Implant médical, dispositif, ou préparation pharmaceutique implantable selon l'une quelconque des revendications précédentes, dans lequel la composition de cellulose non modifiée est l'hydroxyéthyl cellulose.

5. Implant médical, dispositif, ou préparation pharmaceutique implantable selon l'une quelconque des revendications précédentes, dans lequel la composition de cellulose non modifiée est la carboxyméthyl cellulose.

6. Implant médical, dispositif, ou préparation pharmaceutique implantable selon l'une quelconque des revendications précédentes, dans lequel la composition inclut au moins un autre polymère.

7. Implant médical, dispositif, ou préparation pharmaceutique implantable selon la revendication 6, dans lequel le au moins un autre polymère est l'acide hyaluronique.

8. Implant médical, dispositif, ou préparation pharmaceutique implantable selon l'une quelconque des revendications précédentes, qui est un dispositif de prothèse, un dispositif de prothèse synthétique ou de bioprothèse, une suture ou une tubulure.

9. Implant médical, dispositif, ou préparation pharmaceutique implantable selon l'une quelconque des revendications précédentes, dans lequel le dispositif est utilisé pour libérer une substance médicamenteuse choisie dans le groupe constitué par les protéines, les produits pharmaceutiques, les anticorps, les biopolymères, et le polymères synthétiques biologiquement compatibles.

10. Implant médical, dispositif, ou préparation pharmaceutique implantable selon la revendication 9, dans lequel la protéine est un facteur de croissance ou une enzyme.

11. Procédé de préparation d'un dispositif implantable comprenant
la sélection d'une substance médicamenteuse pour utilisation dans le traitement d'un patient souffrant d'un état médical particulier,
la sélection d'une composition de carboxyméthyl cellulose, carboxyméthyl amylose ou hydroxyéthyl cellulose non modifiée ayant un poids moléculaire inférieur à 100 kdaltons qui, par implantation chez un sujet sous forme de composant d'un dispositif implantable, est capable de libérer le médicament dans le patient suivant un profil de libération prédéterminé, et
la préparation d'un dispositif implantable en combinant la substance médicamenteuse et la composition de cellulose.

12. Le procédé selon la revendication 11 incluant additionnellement l'une quelconque des caractéristiques des revendications 2 à 7.
